# EUROPEAN PATENT APPLICATION

(11) **EP 0 769 697 A1**
(43) Date of publication of application: **23.04.1997**
(21) Application number: 96116778.0
(22) Date of filing: 18.10.1996
(51) Int. Cl.: G01N 33/68, G01N 33/543

(54) **Dry test apparatus for glycated albumin determination**

(30) Priority: 18.10.1995 JP 304919/95; 20.10.1995 JP 308101/95
(71) Applicant: KYOTO DAIICHI KAGAKU CO., LTD., Kyoto-shi Kyoto-fu (JP)
(72) Inventor: Yamamoto, Hiroshi, Kyoto Daiichi Kagaku co., Ltd., Minami-ku, Kyoto-shi, Kyoto (JP); Doi, Shigeru, Kyoto Daiichi Kagaku co., Ltd., Minami-ku, Kyoto-shi, Kyoto (JP); Kono, Masao, Kyoto Daiichi Kagaku co., Ltd., Minami-ku, Kyoto-shi, Kyoto (JP)
(74) Representative: Hansen, Bernd, Dr. Dipl.-Chem.

(57) **Abstract**

A dry test apparatus for determining glycated albumin by simultaneously determining albumin and glycated albumin in whole blood by using a developer, which comprises a support having provided thereon a developing layer, a blood cell-separating layer, a reagent layer containing an albumin-staining dye and a glycated albumin-staining dye; a measuring layer having fixed thereto an albumin-binding substance; and a residual liquid-absorbing layer which absorbs a residual developer.

## Description

### FIELD OF THE INVENTION

The present invention relates to a test apparatus for simultaneously measuring albumin and glycated albumin which is glycated protein of albumin in clinical test.

### BACKGROUND OF THE INVENTION

It is known that albumin in serum, or hemoglobin in erythrocytes non-enzymatically react with glucose to produce the corresponding glycated proteins, i.e., glycated albumin or glycated hemoglobin, respectively. Since the concentration of the glycated protein reflects the blood glucose level during the period when proteins are present in the body fluid, measurement of the glycated protein concentration is particularly useful for a *in vitro* diagnosis of diabetes and monitoring diabetes mellitus.

Glycated proteins that reflect a blood glucose level include hemoglobin A_{1c} (HbA_{1c}), which is glycated hemoglobin, glycated albumin, and fructosamine. An HbA_{1c} concentration reflects the blood glucose level during past 1 to 2 months, while glycated albumin and fructosamine concentrations reflect the blood glucose level of past 1 to 2 weeks and therefore tend to show faster response and larger variation than an HbA_{1c} concentration.

In other words, a glycated albumin or fructosamine concentration is more suitable than an HbA_{1c} concentration as an indication for short-term blood glucose control. The concentration of fructosamine is determined by colorimetric analysis based on its reducing character, the measurement is apt to undergo influences of other reducing substances present in the blood, such as bilirubin and ascorbic acid. Furthermore, since the measured fructosamine content is expressed by an absolute value, it is influenced by the amount of proteins in a sample. Therefore, a glycated albumin concentration which is expressed in terms of relative value is deemed to be a more reliable marker than a fructosamine concentration.

In clinical test, a glycated albumin level is generally expressed in terms of a ratio to total albumin, and a glycated albumin level ranging from 11 to 16% is usually taken as a criterion for ascertaining normal. Accordingly, it is required to determine both total albumin and glycated albumin at the same time in order to know the glycated albumin level,

Glycated protein can be determined by electrophoresis, ion-exchange chromatography, high performance liquid chromatography (HALC) making use of affinity to boric acid (e.g., Akazawa et al, *Igaku To Yakugaku,* 29:269-274 (1993); Fujita et al, *Kiki·Shiyaku, 17:689-694* (1994)), enzyme immunoassay, an assay using a binder (U.S. Patent 5,110,745), and the like. of these methods HPLC is used mainly in the field of clinical test. However, HPLC, while reliable in accuracy and separating performance, is time-consuming for measurement per sample and therefore cannot be applied to a large number of samples at a time.

A combination of boric acid affinity and immunoassay has recently been adopted (e.g., Akazawa et al, *Igaku To Yakugaku, 29*:269-274 (1993); Nakano et al, *Kiki·Shiyaku,* 16:1017-1021 (1993); Fujita et al, *Kiki·Shlyaku, 17*:689-694 (1994)), Immunoassay is generally excellent in specificity to glycated protein and suitable for treating a large number of samples.

Furthermore, a boric acid-induced enzyme reagent for detecting glycated proteins has been reported (e.g., Miyake et al, *Igaku To Yakugaku, 28*:871-875 (1992); Miyake et al, *Igaku To Yakugaku, 28*:876-882 (1992); Kanatsuna et al, *The Clinical Report, 26*:5585-5589 (1992)).

Also, monoclonal antibodies specific for human glycated albumin have been reported (e.g., EP-B-0257421, U.S. Patents 5,173,422, 5,223,392 and 5,225,354).

Under the present situation, the above-mentioned HPLC and immunoassay are carried out in clinical laboratories or large hospitals by moans of relatively large-scaled equipment. It has therefore been demanded to develop a method for determining glycated protein by which doctors of small- to medium-sized hospitals, general practioners, and even patients themselves can examine the sample with simple and small equipment in a short time. No report has been made on a method responding this demand.

For determination of glycated albumin, serum or plasma is used as a specimen so that a blood sample must be pretreated for removing blood cells to obtain serum or plasma. Separation of serum is accompanied by not only complicated operation but a danger of infection called biohazard. The difficulty in serum separation has also been a hinderance to realization of glycated protein determination with a dry test apparatus.

### SUMMARY OF THE INVENTION

An object of the present invention is to provide a dry test apparatus for simultaneously determining total albumin and glycated albumin to know a glycated albumin level, with which doctors of small- to medium-sized hospitals, general practioners, and even patients themselves can examine a blood sample with simple and small equipment for photometry without requiring pretreatment for serum separation.

This and other objects of the present invention have been accomplished by a dry test apparatus for determining glycated albumin by simultaneously determining albumin and glycated albumin in whole blood by using a developer, which comprises:
(A) a support having provided thereon a developing layer;
(B) a blood cell-separating layer;
(C) a reagent layer containing an albumin-staining dye (hereinafter often referred to dye 1) and a glycated albumin-staining dye (hereinafter often referred to dye 2);
(D) a measuring layer having fixed thereto an albumin-binding substance; and
(E) a residual liquid-absorbing layer which absorbs a residual developer,
wherein said blood cell-separating layer, said reagent layer, said measuring layer, and said residual liquid-absorbing layer are arranged sequentially on said developing layer (hereinafter referred to as a first embodiment).

In the dry test apparatus according to the first embodiment of the present invention, the albumin-staining dye and the glycated albumin-staining dye preferably have different absorption wavelengths or different fluorescence emission wavelengths.

Furthermore, this and other objects of the present invention have been accomplished by a dry test apparatus for determining glycated albumin in whole blood by using a developer, which is totally made up of a material capable of spreading a liquid therethrough and comprises:
(A) a support having a through-hole or a light-transmitting area enabling optical measurement;
(B) a measuring layer having fixed thereto an albumin-binding substance, said measuring layer being laminated on said support so as to cover said through-hole or light-transmitting area;
(C) a developing layer laminated on said measuring layer;
(D) a reagent layer containing an albumin-staining dye and a glycated albumin-staining dye, said reagent layer being laminated on said developing layer;
(E) a blood cell-separating layer comprising a blood cell-separating membrane, said blood cell-separating layer being laminated on said reagent layer; and
(F) a residual liquid-absorbing layer which absorbs a residual developer, said residual liquid-absorbing layer being provided so as to surround the periphery of said measuring layer,
wherein said albumin-staining dye and said glycated albumin-staining dye have different optical characteristics (hereinafter referred to as a second embodiment).

The test apparatus according to the second embodiment is of the type in which a support has provided thereon a single laminated structure and which makes it possible to determine glycated albumin and albumin simultaneously in a single measuring layer.

Moreover, this and other objects have been accomplished by a dry test apparatus for determining glycated albumin in whole blood by using a developer, which is totally made up of a material capable of spreading a liquid therethrough and comprises:
(A) a support having two through-holes or light-transmitting areas enabling optical measurement;
(B) a laminated structure comprising:
   (a) a measuring layer having fixed thereto an albumin-binding substance, the measuring layer being laminated on the support so as to cover one of said through-holes or light-transmitting areas;
   (b) a developing layer laminated on said measuring layer;
   (c) a reagent layer containing an albumin-staining dye, said reagent layer being laminated on the developing layer;
   (d) a blood cell-separating layer comprising a blood cell-separating membrane, said blood cell-separating layer being laminated on said reagent layer; and
   (e) a residual liquid-absorbing layer which absorbs a residual developer, said residual liquid-absorbing layer being provided so as to surround the periphery of said measuring layer; and
(C) a laminated structure comprising:
   (a) a measuring layer having fixed thereto an albumin-binding substance or a glycated albumin-binding substance, said measuring layer being laminated on said support so as to cover the other through-hole or light-transmitting area;
   (b) a developing layer laminated on said measuring layer;
   (c) a reagent layer containing a glycated albumin-staining dye, the reagent layer being laminated on said developing layer;
   (d) a blood cell-separating layer comprising a blood cell-separating membrane, said blood cell-separating layer being laminated on the reagent layer; and
   (e) a residual liquid-absorbing layer which absorbs a residual developer, said residual liquid-absorbing layer being provided so as to surround the periphery of said measuring layer (hereinafter referred to as a third embodiment).

The test apparatus according to the third embodiment is of the type in which a support has provided thereon two laminated structures; one for determining glycated albumin and the other for determining albumin (and glycated albumin). In the third embodiment, because the albumin-staining dye and the glycated albumin-staining dye are to be detected in the respective laminated structures, they may have the same optical characteristics.

### BRIEF DESCRIPTION OF THE DRAWINGS

Figs. 1A through 3B are each a plane view and a cross-sectional view of an example of the test apparatus according to the first embodiment of the present invention (designated test apparatuses 11, 12, and 13, respectively).

Figs. 4A and 4B are a plane view and a cross-sectional view of a modification of test apparatus 11 shown in Fig. 1 (designated test apparatus 11').

Pig. 5 is a cross section of test apparatus 21 according to the second embodiment of the present invention.

Fig, 6 is a cross section of test apparatus 31 according to the third embodiment of the present invention.

### DETAILED DESCRIPTION OF THE INVENTION

In the present invention of the test apparatus according to the first, second and third embodiments of the present invention, glycated albumin determination can be carried out simply by dropping a blood sample on the test apparatus or apparatus and developing the sample with a developer, whereby albumin and glycated albumin are stained with a dye(s) and then trapped in the measuring layer. Each of the albumin and glycated albumin thus stained and trapped is optically measured with equipment for photometry. A ratio of glycated albumin to albumin is calculated from the measurements.

The test apparatuses according to the second and third embodiments of the present invention are advantageous in that a blood sample can be developed evenly because the sample is developed downward by gravity.

Applicable blood samples include previously separated serum or plasma as well as whole blood. Although a blood cell-separating layer is not necessary in using serum or plasma, the test apparatuses of the present invention are designed to examine whole blood so that even a patient may make a determination by himself at home.

If desired, the reagent layer may contain a protein denaturant which denaturalizes the albumin and glycated albumin to expose their epitopes as much as possible thereby to facilitate binding to the respective antibodies.

JP-A-3-87809 discloses a method of determining glycated albumin comprising trapping both albumin and glycated albumin by a solid phase antibody and labeling only glycated albumin with a boric acid derivative ("JP-A" as used herein means an "unexamined Japanese patent application"). In this method, a sample in excess over the known amount of the antibody in the solid phase (i.e., glycated albumin-containing albumin) is applied, and a given amount of albumin is trapped without labeling albumin. The trapping capacity of the solid phase antibody must be always constant. Because the trapping capacity is liable to be affected by various factors, tending to cause error, preparation and preservation of the solid phase should be controlled strictly and also need skill. Additionally, a large volume of a sample is required.

In the determination of glycated hemoglobin, since hemoglobin itself is red, only glycated hemoglobin should be labeled. Albumin being colorless, on the other hand, both albumin and glycated albumin must be labeled with respective labeling substances. Hence, in the present invention, albumin is labeled so as to determine total albumin inclusive of glycated albumin.

Dyes are used as a labeling substance in the present invention because dyes are far less expensive and more stable than enzymes or radioactive substances and also because two different dyes can be distinguished only if photometric equipment enabling dual wavelength spectrophotometry is available, making it possible to determine albumin and glycated albumin. Furthermore, dyes are of great advantage in that many of them emit fluorescence. The terminology "optical characteristics" as used herein for dyes means absorption wavelengths and fluorescence emission wavelengths.

### PREFERRED EMBODIMENTS OF THE INVENTION

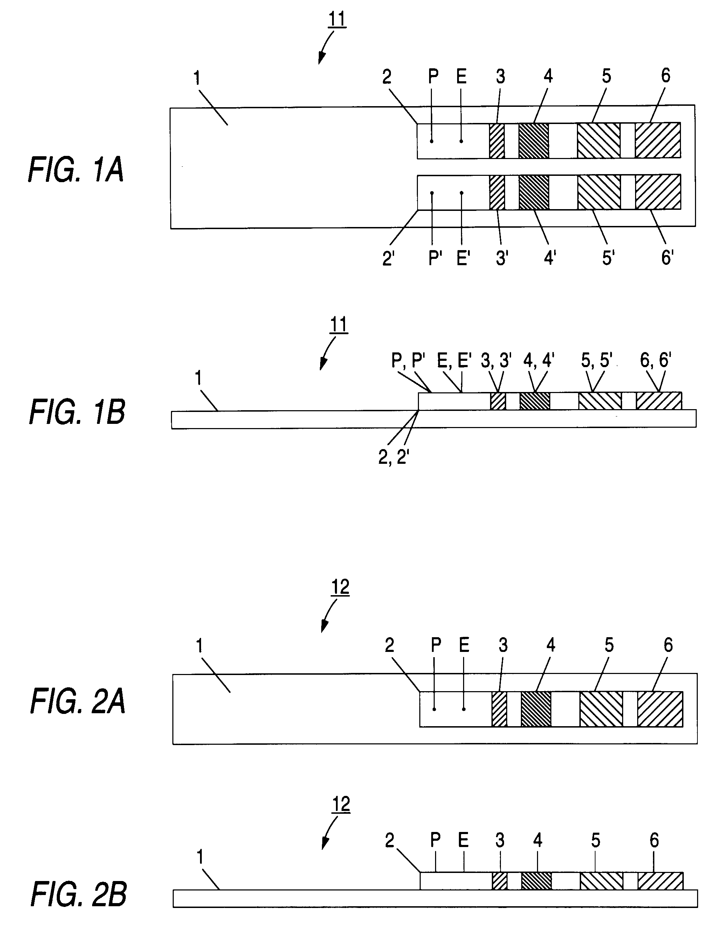

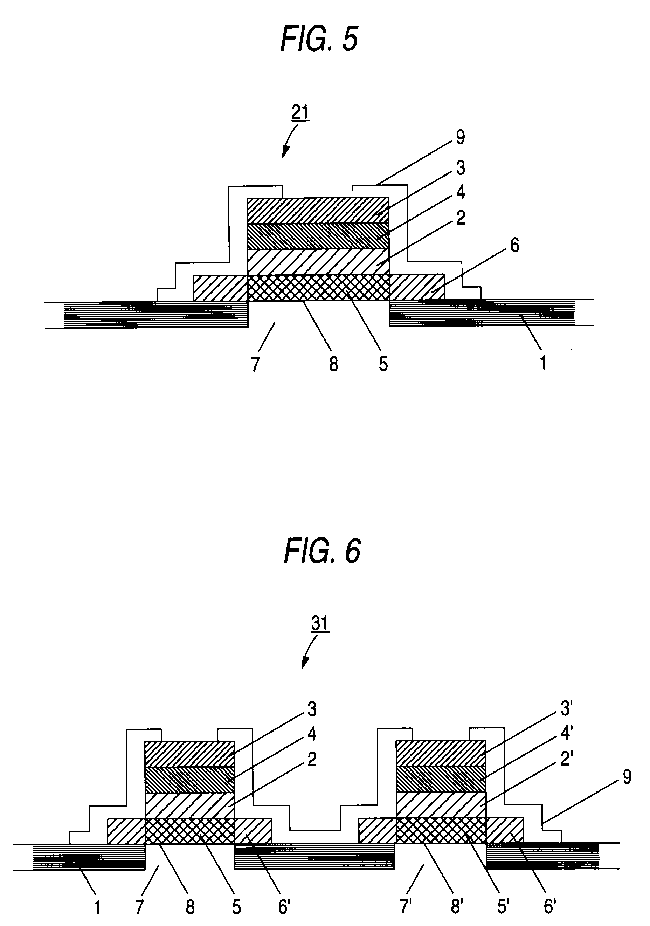

The embodiments of the present invention will be illustrated by referring to the accompanying drawings. Figs. 1A through 4B are each a plane view and a cross-sectional view showing a test apparatus according to the first embodiment, and Fig. 5 and Fig. 6 are each a cross section showing a test apparatus according to the second and third embodiments of the present invention, respectively. The test apparatuses of the present invention are essentially characterized in that layers capable of transferring liquid are laminated on one another to form a laminated structure through which liquid develops downward.

### Test Apparatus 11

Figs. 1A and 1B show a plane view and a cross-sectional view of an example of the test apparatus according to the first embodiment, which comprises support having provided thereon two developing layers 2 and 2' made of the same material capable of spreading a developer. Developing layer 2 has position P for developing initiation, position E for sample application, blood cell-separating layer 3 comprising a blood cell-separating membrane, reagent layer 4 coated with dye 1 including a denaturant optionally, measuring layer 5 having fixed thereto an albumin-binding substance, and residual liquid-absorbing layer 6 which absorbs a residual developer in this order. Developing layer 2' has position P' for developing initiation, position E' for sample application, blood cell-separating layer 3' comprising a blood cell-separating membrane, reagent layer 4' coated with dye 2 including a denaturant optionally, measuring layer 5' having fixed thereto an albumin-binding substance or a substance which binds to glycated albumin without binding to non-glycated albumin (hereinafter referred to an "glycated albumin-binding substance"), and residual liquid-absorbing layer 6' which absorbs a residual developer in the order listed.

When the glycated albumin-binding substance is used in measuring layer 5', a dye which stains both albumin and glycated albumin may be used in reagent layer 4' instead of dye 2.

Test apparatus 11 has two developing layers, and albumin and glycated albumin pass different reagent layers and are bound in different measuring layers.

A blood sample is dropped on position E of test apparatus 11, and a developer is then applied to position P, whereby the blood sample is developed, and blood cells are removed by the blood cell-separating membrane of blood cell-separating layer 3. Albumin in the blood is denaturalized by the denaturant optionally and stained with dye 1 in reagent layer 4. The thus stained albumin is trapped in measuring layer 5, while the other substances are absorbed by residual liquid-absorbing layer 6. Glycated albumin also may be stained with dye 1, and be trapped in measuring layer 5.

On the other hand, the same amount of a blood sample is dropped on position E', and a developer is then applied to position P', whereby the blood sample is developed, and blood cells are removed by the blood cell-separating membrane of blood cell-separating layer 3'. Glycated albumin in the blood is denaturalized by the denaturant optionally and stained with dye 2 in reagent layer 4'. The thus stained glycated albumin is trapped in measuring layer 5', while the other substances are absorbed by residual liquid-absorbing layer 6'.

The color of the albumin-dye 1 complex or the albumin-and glycated albumin-dye 1 complex trapped in measuring layer 5 and that of the glycated albumin-dye 2 complex trapped in measuring layer 5' are optically detected to obtain the concentrations of the respective dyes. The amounts of albumin and glycated albumin are obtained from the dye concentrations, from which is calculated the glycated albumin to total albumin (glycated albumin and non-glycated albumin) ratio.

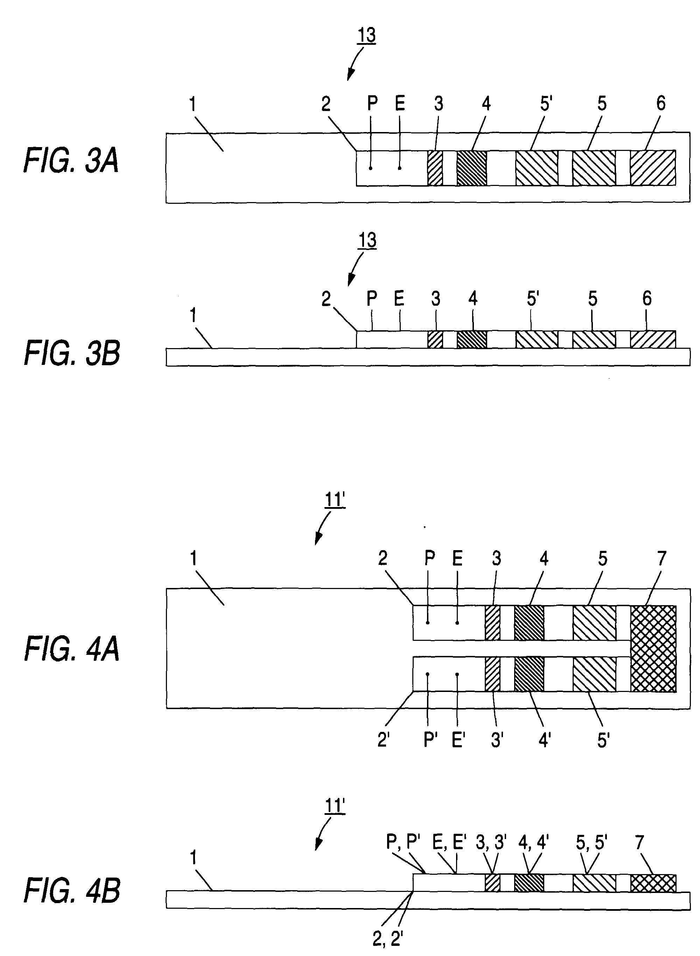

As shown in Figs. 4A and 4B (test apparatus 11'), the residual liquid-absorbing layers 6 and 6' separately provided in test apparatus 11 may be replaced with a single residual liquid-absorbing layer 7 which can be shared by the two developing layers.

In test apparatus 11 shown in Figs. 1A and 1B, developing layers 2 and 2' are arranged close to one end of support 1 in order for a measurer may pick the other end of the support between his fingers. As a matter of course, the developing layers may be arranged at an arbitrary position of support 1. This also applies to test apparatuses 12, 13, 21 and 31 hereinafter illustrated.

Materials used in the present invention are selected from conventional ones.

### Support:

The support for use in test apparatus 11 includes a film, a sheet or a plate made mainly of polystyrene, polyester, cellulose acetate, and the like. The support may be either light-transmitting or non-light-transmitting. Whore photometry after sample development is conducted from the reverse side of the support, it is desirable to use a transparent support, for example, a transparent polyethylene terephthalate (PET) support is preferred.

### Developing Layer:

The developing layer includes a filter paper-like or porous matrix made up mainly of cellulose, a cellulose derivative (e.g., cellulose acetate, nitrocellulose), glass fiber, and the like. A developing layer cut into a strip as illustrated in Figs. 1A and 1B is fixed physically onto the support with a double-sided adhesive tape, an adhesive or a hot-melt adhesive. The width and length of the strip are decided in accordance with known chromatographic techniques.

### Blood Cell-Separating Membrane:

The blood cell-separating membrane for use in the present invention includes a filter membrane mainly comprising polytetrafluoroethylene, polysulfone, polypropylene, polyvinylidene difluoride, cellulose mixed esters, and the like.

### Denaturant:

The denaturant for use in the present invention includes a surfactant, an acid, saponin, and guanidine or a salt thereof. The denaturant is used for denaturalizing the albumin and glycated albumin to expose their epitopes as much as possible thereby to facilitate binding to the antibody. While a denaturant is not always needed for some antibodies, addition of a denaturant gives no adverse effect.

### Absorbent:

The absorbent constituting the residual liquid-absorbing layer is required to have a sufficient pore volume for absorbing the developer after development. Useful absorbents include a filter paper-like or porous matrix made mainly of cellulose, a cellulose derivative (e.g., cellulose acetate or nitrocellulose), glass fiber, and the like.

### Developer:

The developer which can be used for developing a blood sample preferably includes those commonly employed in immunoassay or chromatography, such as a buffer solution containing trace amounts of protein, a salt, a chelate compound, a surfactant, and the like. A 0.1M phosphate buffer solution containing 0.1% bovine serum albumin, 0.9% sodium chloride, 0.01% EDTA, and 0.01% Triton X-100 can be mentioned as a typical example of the developer.

### Dye:

Dye 1 which can be used for staining albumin includes those having specific affinity for albumin but no affinity for other proteins. Dye 1 includes dyes which stains albumin without staining glycated albumin, and dyes which stains both albumin and glycated albumin.

For example, indicators, such as Bromocresol Purple, Bromophenol Blue, Bromocresol Green, and Cibacron Blue F3G-A, are preferred.

Those dyes which have no particular specific affinity for albumin can also be used via an anti-albumin antibody, an ion exchanger described in the measuring layer below, or a combination thereof. For example, anthraquinone dyes, azo dyes, amine dyes, triazine dyes, phycoerythrin dyes, cumarin dyes, acridine dyes, rhodamine dyes, fluorescent dyes, and phthalocyanine dyes bound to an anti-albumin antibody can be used as dye 1. of the dyes enumerated above, those emission may be detected through measurement of the fluorescence emission wavelengths. Dyes specifically staining albumin are very useful for their inexpensiveness, stability, and also because they can be bound to albumin without being mediated by an antibody.

Dye 2 which can be used for staining glycated albumin includes those having specific affinity for glycated albumin. Dyes having no glycated albumin specificity are used via an anti-glycated albumin antibody, a dihydroxyboric acid derivative, an ion exchanger described in the measuring layer below, or a combination thereof. For example, anthraquinone dyes, azo dyes, azine dyes, triazine dyes, phycoerythrin, cumarin dyes, acridine dyes, rhodamine dyes, fluorescent dyes, and phthalocyanine pigments bound to a dihydroxyboric acid derivative or an anti-glycated albumin antibody can be used. Xylene cyanolboric acid and a dimethylaminonaphthalenesulfonyl anti-glycated albumin antibody are particularly suitable. The anti-glycated albumin antibody is selected from those which recognize the glucose moiety of glycated albumin. If the ion exchanger is used, the teat apparatus must be composed not so as to combine the dye-2 and ion exchanger complex with albumin.

The anti-albumin antibody and the anti-glycated albumin antibody may be either a polyclonal antibody or a monoclonal antibody. Not only antibody molecules but antibody active fragments obtained by enzymatically treating the antibody molecules, such as Fab, Fab', and (Fab')₂, can be used.

Where a combination of a dye and a dihydroxyboric acid derivative is used, because an anti-glycated albumin antibody has a glucose moiety, it is preferable to previously remove the glucose chain by enzymatic treatment with glycopeptidase or N-glycase or to use the above-mentioned Fab, Fab' or (Fab')₂ fragment.

### Measuring Layer:

The measuring layers of test apparatus 11 comprises a matrix having fixed thereto an albumin-binding substance through chemical bending. Examples of the albumin-binding substance include anti-albumin antibodies, ion exchangers, and combinations thereof. The albumin-binding substance for use in the present invention can bind to both albumin and glycated albumin. The anti-albumin antibody may be a polyclonal antibody or a monoclonal antibody, and be treated enzymatically as described above. Suitable ion exchangers include diethylaminoethyl cellulose and carboxymethylated polyvinyl alcohol.

Measuring layer 5' for trapping glycated albumin may comprise an albumin-binding substance or a glycated albumin-binding substance. Examples of the glycoalbumin-binding substance include anti-glycated albumin antibodies, dihydroxyboric acid derivatives, and combinations thereof. These substances do not bind to albumin, but binds to glycated albumin because they recognize the glucose moiety of glycated albumin. The anti-glycated albumin antibody may be a polyclonal antibody or a monoclonal antibody, and be treated enzymatically as described above.

Where dyes 1 and 2 require mediation by an antibody, and the albumin-binding substance in the measuring layer contains an antibody, these antibodies should have different epitopes and be bound to an antigen simultaneously.

### Constitution:

The above-described various layers are provided on the developing layer by impregnating, coating or binding the developing layer with the respective reagent by any method well known in the art. For example, a substance to be applied is dissolved in an impregnating solution, and the solution is applied to the developing layer by impregnation or by spraying with an ink jet printer, followed by drying. Because the reagent in the reagent layer is ought to be oozed out from the initial position and developed with a developer, it is applied simply by impregnation or coating. On the other hand, the specifically binding substance in the measuring layer must be fixed to the developing layer by chemical bonding so as not to immigrate from its position (see *KOSOMEN-EKI SOKUTEIHO,* Igakushoin (1987)).

It is essentially required that dyes 1 and 2, each selected from amongst the aforementioned dyes, be combined so that they stain albumin and glycated albumin independently and specifically. A suitable example of such a combination is Bromocresol Purple as dye 1 and xylene cyanolboric acid as dye 2. Since test apparatus 11 has a measuring layer exclusive for albumin determination and a measuring layer exclusive for glycated albumin determination, dyes 1 and 2 may be of the same kind or may have the same absorption wavelength and yet are distinguishable.

### Test Apparatus 12

Figs. 2A and 2B show a plane view and a cross-sectional view of another example of the test apparatus according to the first embodiment of the present invention, which comprises support 1 having provided thereon a single developing layer 2.

Developing layer 2 has position P for developing initiation, position E for sample application, blood cell-separating layer 3 comprising a blood cell-separating membrane, reagent layer 4 coated with dyes 1 and 2 including a donaturant optionally, measuring layer 5 having fixed thereto an albumin-binding substance, and residual liquid-absorbing layer 6 which absorbs a residual developer in the order listed.

Test apparatus 12 has a single developing layer, and albumin and glycated albumin pass a single reagent layer and are trapped in a single measuring layer.

A blood sample is dropped on position E of test apparatus 12, and a developer is then applied to position P, whereby the blood sample is developed, and blood cells are removed by the blood cell-separating membrane of blood cell-separating layer 3. Albumin and glycated albumin in the blood are denaturalized by the denaturant in reagent layer 4 optionally, and albumin is stained with dye 1, and glycated albumin is stained with dyes 1 and 2 or dye 2. The thus stained albumin and stained glycated albumin are trapped in measuring layer 5, while the other substances are absorbed by residual liquid-absorbing layer 6.

The color of dye 1 in the albumin-dye 1 complex or in the albumin-dye 1 complex and the glycoalbumin-dyes 1 and 2 complex, and that of dye 2 in the glycoalbumin-dye 2 complex or in the glycoalbumin-dyes 1 and 2 complex trapped in measuring layer 5 are optically detected separately to obtain the concentrations of the respective dyes. The amounts of albumin and glycated albumin are obtained from the dye concentrations, from which is calculated the ratio of glycated albumin to total albumin.

Having a single developing layer, test apparatus 12 is simpler in structure and less costly than test apparatus 11 having two developing layers. Glycated albumin can be determined by dropping a blood sample only once, so that the measurement is simpler.

The materials constituting the support, developing layer, and various layers of test apparatus 12 are the same as in test apparatus 11. However, where a staining dye is used as dye 1, dye 1 may stain not only albumin but glycated albumin. In this case, glycated albumin is stained by both dyes 1 and 2. Therefore, dyes 1 and 2 should be selected so that they can be distinguished optically, i.e., should have different absorption wavelengths, in order to observe dyes 1 and 2 in one measuring layer. This is the difference between test apparatuses 11 and 13 of the first embodiment. It is a matter of course that dye 2 should label only glycated albumin.

Where dyes 1 and 2 require mediation by an antibody, and the specifically binding substance in the measuring layer contains an antibody, these antibodies should have different epitopes and be bound to an antigen simultaneously.

### Test Apparatus 13

Figs. 3A and B show a plane view and a cross-sectional view of still another example of the test apparatus according to the first embodiment of the present invention, which comprises support 1 having provided thereon a single developing layer 2.

Developing layer 2 has position P for developing initiation, position E for sample application, blood cell-separating layer 3 comprising a blood cell-separating membrane, reagent layer 4 coated with dyes 1 and 2 including a denaturant optionally, measuring layer 5' having fixed thereto an glycated protein-binding substance, measuring layer 5 having fixed thereto an albumin-binding substance, and residual liquid-absorbing layer 6 which absorbs a residual developer in the order listed.

Test apparatus 13 has a single developing layer, and albumin and glycated albumin pass a single reagent layer and are trapped in different measuring layers.

A blood sample is dropped on position E of test apparatus 13, and a developer is then added to position P, whereby the blood sample is developed, and blood cells are removed by the blood cell-separating membrane of blood cell-separating layer 3. Albumin and glycated albumin in the blood are denaturalized by the denaturant in reagent layer 4 optionally, and albumin is stained with dye 1, and glycated albumin is stained with dyes 1 and 2 or dye 2. The thus stained glycated albumin is trapped in measuring layer 5', and the thus stained albumin is trapped in measuring layer 5, while the other substances are absorbed by residual liquid-absorbing layer 6.

The color of dye 2 in the glycated albumin-dyes 1 and 2 complex or in the glycated albumin-dye 2 complex, and that of dye in the albumin-dye 1 complex or in the albumin-dye 1 complex and glycated albumin-dyes 1 and 2 complex trapped in measuring layers 5' and 5, respectively, are optically detected separately to obtain the concentrations of the respective dyes. The amounts of albumin and glycated albumin are obtained from the dye concentrations, from which is calculated the ratio of glycated albumin to total albumin.

Test apparatus 13 has the same advantage as test apparatua 12. In addition, since test apparatus 13 has a measuring layer exclusive for albumin determination and a measuring layer exclusive for glycated albumin determination, dyes 1 and 2 can be distinguished even if they are of the same kind or have the same absorption wavelength. Dye 1 may be either a dye which specifically stains albumin alone or a dye which stains both albumin and glycated albumin; provided that dye 2 should label only glycated albumin.

If one of the measuring layers (layer 5') has fixed therein an anti-glycated albumin antibody while the other measuring layer (layer 5) has fixed therein an anti-albumin antibody, only a dye which stains both albumin and glycated albumin simultaneously may be employed. In this case, the dye-staining albumin and the dye-staining glycated albumin have utterly the same absorption wavelength and yet can be distinguished because of the two separate measuring layers.

### Test Apparatus 21

Fig. 5 shows a cross-sectional view of an example of the test apparatus according to the second embodiment of the present invention, which comprises support 1 having provided thereon a single laminated structure (a layered structure).

Test apparatus 21 comprises support 1 having through-hole 7 having laminated on the through-hole thereof transparent layer 8, measuring layer 5 having fixed thereto an albumin-binding substance, developing layer 2, reagent layer 4 having carried thereon dyes 1 and 2 including a denaturant optionally, and blood cell-separating layer 3 comprising a blood cell-separating membrane in the order listed, measuring layer 5 being surrounded by residual liquid-absorbing layer 6 which absorbs a residual developer, to form a laminated structure. The laminated structure is covered with cover 9 having a through-hole through which a blood sample and a developer are supplied.

On dropping a blood sample on blood cell-separating layer 3 and then adding a developer to that layer, the blood sample is developed, and the blood cells are separated by the blood cell-separating membrane. Albumin and glycated albumin in the plasma are denaturalized by the denaturant in reagent layer 4 optionally, where albumin is stained with dye 1, while glycated albumin is stained with dyes 1 and 2 or dye 2. Albumin stained with dye 1 and glycated albumin stained with dyes 1 and 2 or dye 2 are trapped in measuring layer 5, while the other substances are absorbed by residual liquid-absorbing layer 6.

The color of the albumin-dye 1 complex or the albumin- and glycated albumin-dye 1 complex and that of glycated albumin-dye 2 complex both trapped in measuring layer 5 are optically detected separately to obtain the concentrations of the respective dyes. The amounts of albumin and glycated albumin are obtained from the dye concentrations, from which is calculated the ratio of glycated albumin to albumin.

The support, blood cell-separating membrane, absorbent, developer, dyes 1 and 2, denaturant, measuring layer and the like which can be used in the preparation of test apparatus 21 and in the determination using test apparatus 21 are the same as those used as for test apparatus 11.

where a non-light-transmitting support is used, a through-hole is made as in Fig. 5.

The transparent layer used in test apparatus 21 includes a film or a sheet made mainly of polyester, cellulose acetate, polycarbonate, polystyrene, and the like. The transparent layer is provided to hold the measuring layer covering the through-hole of the support. Therefore, it is not necessary where the measuring layer itself has strength to some extent or where the support itself is light-transmitting. Assuming that the support is non-light-transmitting in Fig. 5, the support has a through-hole, and a transparent layer is provided beneath the measuring layer.

The material of cover 9 is not particularly limited and may be the same as used for the support, i.e., a film or a sheet made mainly of polyester, cellulose acetate, polycarbonate, polystyrene, and the like. Cover 9 is for ensuring laminating of the layers. Where the layers are fixed together with a small amount of an adhesive, cover 9 is unnecessary.

Each layer constituting test apparatus 21 is prepared by impregnating or coating a filter paper-like or porous matrix made mainly of cellulose, a cellulose derivative (e.g., cellulose acetate, nitrocellulose), glass fiber, and the like with the respective reagent and the like in the same manner as in test apparatus 11.

In test apparatus 21, stained-albumin and stained-glycated albumin are measured in the same layer. In order to clearly distinguish between dyes 1 and 2 in a single measuring layer, dyes 1 and 2 must have different optical characteristics such as absorption wavelengths and fluorescence emission wavelengths.

Having a single laminated structure, test apparatus 21 is simpler in structure and less costly than test apparatus 31 hereinafter described which has two laminated structures. Glycated albumin can be determined by dropping a blood sample only once, so that the measurement is simpler.

### Test Apparatus 31

Fig. 6 shows a cross-sectional view of an example of the tort apparatus according to the third embodiment of the present invention, which comprises support 1 having provided thereon two laminated structure (layered structures).

Test apparatus 31 comprises support 1 having two through-holes 7 and 7' having laminated on each through-hole thereof transparent layer 8 or 8', measuring layer 5 or 5' having fixed thereto an albumin-binding substance, developing layer 2 or 2', reagent layer 4 having carried thereon dye 1 or reagent layer 4' having carried thereon dye 2 including a denaturant optionally, and blood cell-separating layer 3 or 3' comprising a blood cell-separating membrane in the order listed, each of measuring layers 5 and 5' being surrounded by residual liquid-absorbing layer 6 or 6', respectively, which absorbs a residual developer, to form the respective laminated structures. The two laminated structures are covered with cover 9 having a through-hole on each laminated structure, through which a sample and a developer are supplied.

In measuring layer 5, albumin (and glycated albumin) are stained. In measuring layer 5', only glycated albumin is stained. Thus, a ratio of glycated albumin to total albumin can be obtained.

While the materials constituting each layer of test apparatus 31 are the same as in test apparatus 21, test apparatus 31 is advantageous in that dyes 1 and 2 can be distinguished from each other even if they have the same optical characteristics and are undistinguishable optically because they are individually observed in separate measuring layers.

Where dyes 1 and 2 require mediation by an antibody, and the albumin- and/or glycated albumin-binding substance in the measuring layer contains an antibody, these antibodies should have different epitopee and be bound to an antigen simultaneously, as in the case with test apparatus 21.

As a modification of test apparatus 31, measuring layer 5 may have fixed therein an albumin-binding substance, while measuring layer 5' may have fixed therein a glycated albumin-binding substance. In this case, measuring layer 5 traps albumin and glycated albumin, while measuring layer 5' traps only glycated albumin, thereby enabling calculation of a ratio of glycated albumin to total albumin. In this modified apparatus, too, a dye which stains both albumin and glycated albumin may be used instead of dye 2. In this case, this dye and dye 1 and may be of the same kind or may have the same optical characteristics and are yet distinguishable.

The present invention has been described by referring to particular examples thereof, it should be understood that the present invention embraces various modifications and changes within its spirit and scope. That is, the structure of the test apparatuses hereinabove described may be altered according to the end use. For instance, a support is not always necessary, and part of a support may serve as a developing layer or a laminated structure.

The present invention provides novel and practical test apparatuses for simultaneously determining glycated albumin and albumin, which can be handled easily and are therefore suitable to domestic use.

While the invention has been described in detail and with reference to specific examples thereof, it will be apparent to one skilled in the art that various changes and modifications can be made therein without departing from the spirit and scope thereof.

## Claims

1. A dry test apparatus for determining glycated albumin by simultaneously determining albumin and glycated albumin in whole blood by using a developer, which comprises:
(A) a support having provided thereon a developing layer;
(B) a blood cell-separating layer;
(C) a reagent layer containing an albumin-staining dye and a glycated albumin-staining dye;
(D) a measuring layer having fixed thereto an albumin-binding substance; and
(E) a residual liquid-absorbing layer which absorbs a residual developer,
wherein said blood cell-separating layer, said reagent layer, said measuring layer, and said residual liquid-absorbing layer are arranged sequentially on said developing layer.

2. The dry test apparatus as claimed in claim 1, wherein said albumin-staining dye and said glycated albumin-staining dye have different absorption wavelengths or different fluorescence emission wavelengths.

3. The dry test apparatus as claimed in claim I, wherein said albumin-staining dye stains albumin via an anti-albumin antibody, an ion exchanger, or a combination thereof; and said glycated albumin-staining dye stains glycated albumin via an anti-glycated albumin antibody, a dihydroxyboric acid derivative, an ion exchanger or a combination thereof.

4. The dry test apparatus as claimed in claim 1, wherein said albumin-staining dye is a dye capable of specifically staining albumin with no mediation.

5. The dry test apparatus as claimed in claim 4, wherein said albumin-staining dye is selected from Bromocresol Purple, Bromophenol Blue, Bromocresol Green, and Cibacron Blue F3G-A.

6. The dry test apparatus as claimed in claim 1, wherein said albumin-binding substance is an anti-albumin antibody, an ion exchanger, or a combination thereof.

7. The dry test apparatus as claimed in claim 1, wherein said test apparatus has two developing layers, and albumin and glycated albumin pass different reagent layers and are bound in different measuring layers.

8. The dry test apparatus as claimed in claim 7, wherein albumin is trapped in a measuring layer having fixed thereto an anti-albumin antibody, an ion exchanger, or a combination thereof; and glycated albumin is trapped in a measuring layer having fixed thereto an anti-glycated albumin antibody, a dihydroxyboric acid derivative, or a combination thereof.

9. The dry test apparatus as claimed in claim 7, wherein said two developing layers share a single residual liquid-absorbing layer.

10. The dry test apparatus as claimed in claim 1, wherein said test apparatus has a single developing layer, and albumin and glycated albumin pass a single reagent layer and are trapped in a single measuring layer.

11. The dry test apparatus as claimed in claim 1, wherein said test apparatus has a single developing layer, and albumin and glycated albumin pass a single reagent layer and are trapped in different measuring layers.

12. The dry test apparatus as claimed in claim 11, wherein albumin is trapped in a measuring layer having fixed thereto an anti-albumin antibody, an ion exchanger, or a combination thereof; and glycated albumin is trapped in a measuring layer having fixed thereto an anti-glycated albumin antibody, a dihydroxyboric acid derivative, or a combination thereof.

13. The dry test apparatus as claimed in claim 1, wherein said reagent layer further contains a blood denaturant.

14. A dry test apparatus for determining glycated albumin in whole blood by using a developer, which is totally made up of a material capable of spreading a liquid therethrough and comprises:
(A) a support having a through-hole or a light-transmitting area enabling optical measurement;
(B) a measuring layer having fixed thereto an albumin-binding substance, said measuring layer being laminated on said support so as to cover said through-hole or light-transmitting area;
(C) a developing layer laminated on said measuring layer;
(D) a reagent layer containing an albumin-staining dye and a glycated albumin-staining dye, said reagent layer being laminated on said developing layer;
(E) a blood cell-separating layer comprising a blood cell-separating membrane, said blood cell-separating layer being laminated on said reagent layer; and
(F) a residual liquid-absorbing layer which absorbs a residual developer, said residual liquid-absorbing layer being provided so as to surround the periphery of said measuring layer, and said albumin-staining dye and said glycated albumin-staining dye having different optical characteristics.

15. The dry test apparatus as claimed in claim 14, wherein said albumin-staining dye and said glycated albumin-staining dye have different absorption wavelengths or fluorescence emission wavelengths.

16. The dry test apparatus as claimed in claim 14, wherein said albumin-staining dye stains albumin via an anti-albumin antibody, an ion exchanger, or a combination thereof; and said glycated albumin-staining dye stains glycated albumin via an anti-glycated albumin antibody, a dihydroxyboric acid derivative, an ion exchanger, or a combination thereof.

17. The dry test apparatus as claimed in claim 14, wherein said albumin-staining dye specifically stains albumin with no mediation.

18. The dry test apparatus as claimed in claim 17, wherein said first dye is selected from Bromocresol Purple, Bromophenol Blue, Bromocresol Green, and Cibacron Blue F3G-A.

19. The dry test apparatus as claimed in claim 14, wherein said albumin-binding substance is an anti-albumin antibody, an ion exchanger, or a combination thereof.

20. The dry test apparatus as claimed in claim 14, wherein the laminated structure comprising said measuring layer, said developing layer, said reagent layer, said blood cell-separating layer, and said residual liquid-absorbing layer is covered with a cover having one through-hole through which a blood sample and a developer are supplied.

21. The dry test apparatus as claimed in claim 14, wherein said reagent layer further contains a blood denaturant.

22. A dry test apparatus for determining glycated albumin in whole blood by using a developer, which is totally made up of a material capable of spreading a liquid therethrough and comprises:
(A) a support having two through-holes or light-transmitting areas enabling optical measurement;
(B) a laminated structure comprising:
(a) a measuring layer having fixed thereto an albumin-binding substance, the measuring layer being laminated on the support so as to cover one of said through-holes or light-transmitting areas;
(b) a developing layer laminated on said measuring layer;
(c) a reagent layer containing an albumin-staining dye, said reagent layer being laminated on the developing layer;
(d) a blood cell-separating layer comprising a blood cell-separating membrane, said blood cell-separating layer being laminated on said reagent layer; and
(e) a residual liquid-absorbing layer which absorbs a residual developer, said residual liquid-absorbing layer being provided so as to surround the periphery of said measuring layer; and
(C) a laminated structure comprising:
(a) a measuring layer having fixed thereto an albumin-binding substance or a glycated albumin-binding substance, said measuring layer being laminated on said support so as to cover the other through-hole or light-transmitting area;
(b) a developing layer laminated on said measuring layer;
(c) a reagent layer containing a glycated albumin-staining dye, the reagent layer being laminated on said developing layer;
(d) a blood cell-separating layer comprising a blood cell-separating membrane, said blood cell-separating layer being laminated on the reagent layer; and
(e) a residual liquid-absorbing layer which absorbs a residual developer, said residual liquid-absorbing layer being provided so as to surround the periphery of said measuring layer.

23. The dry test apparatus as claimed in claim 22, wherein said albumin-staining dye stains albumin via an anti-albumin antibody, an ion exchanger, or a combination thereof; and said glycated albumin-staining dye stains glycated albumin via an anti-glycated albumin antibody, a dihydroxyboric acid derivative, an ion exchanger, or a combination thereof.

24. The dry test apparatus as claimed in claim 22, wherein said albumin-staining dye specifically stains albumin with no mediation.

25. The dry test apparatus as claimed in claim 24, wherein said albumin-staining dye is selected from Bromocresol Purple, Bromophenol Blue, Bromocresol Green, and Cibacron Blue F3G-A.

26. The dry test apparatus as claimed in claim 22, wherein said albumin-binding substance is an anti-albumin antibody, an ion exchanger, or a combination thereof.

27. The dry test apparatus as claimed in claim 22, wherein said glycated albumin-binding substance is an anti-glycated albumin antibody, a dihydroxyboric acid derivative, or a combination thereof.

28. The dry test apparatus as claimed in claim 22, wherein the laminated structure comprising said measuring layer, said developing layer, said reagent layer, said blood cell-separating layer, and said residual liquid-absorbing layer is covered with a cover having one through-hole through which a blood sample and a developer are supplied.

29. The dry test apparatus as claimed in claim 22, wherein said reagent layer further contains a blood denaturant.
